Europäisches Patentamt

European Patent Office

Office européen des brevets

(19)

(11) Publication number: **0 222 994**
**B1**

# EUROPEAN PATENT SPECIFICATION

(12)

(45) Date of publication of the patent specification:
31.10.90

(51) Int. Cl.⁵: **G01N 33/00, G01N 33/20**

(21) Application number: **86111873.5**

(22) Date of filing: **27.08.86**

(54) **Gas analyzer for the simultaneous measurement of a plurality of ingredients.**

(30) Priority: **19.10.85 JP 235943/85**

(43) Date of publication of application:
**27.05.87 Bulletin 87/22**

(45) Publication of the grant of the patent:
**31.10.90 Bulletin 90/44**

(84) Designated Contracting States:
**DE FR GB IT SE**

(56) References cited:
**EP-A- 0 024 566**
**DE-A- 2 449 025**
**DE-A- 2 845 426**
**GB-A- 2 119 088**

**American Laboratory, February 1985, pages 69-81, R. A. Nodkaruis "Evaluation of an elemental analyzer"**

(73) Proprietor: **HORIBA, LTD., 2 Miyanohigashi-machi Kissyoin, Minami-ku Kyoto(JP)**

(72) Inventor: **Tsuji, Katsuya, 3-3, 3 Chome Mizukidori, Hyogo-ku Kobe(JP)**
Inventor: **Tanimoto, Masahiro, 559-28, Hazukashi-Hishikawa-cho, Fushimi-ku Kyoto(JP)**
Inventor: **Hirano, Akihiro, 76-2, Uchihata, Kitagawara Yamashiro-cho, Sohraku-gun Kyoto(JP)**
Inventor: **Yamada, Takeshi, 11-5, 3 Chome Kitaoji, Ohtsu-city Shiga-prefecture(JP)**

(74) Representative: **TER MEER - MÜLLER - STEINMEISTER & PARTNER, Mauerkircherstrasse 45, D-8000 München 80(DE)**

## Description

The present invention relates to a gas analyzer for the simultaneous measurement of at least two kinds of gas contained in a sample gas.

When using such an analyzer, the problems arises how to lead the gas to the different detectors. Three possibilities exist: The first is to send the gas to detectors which are arranged in series one behind the other. The second is to lead the gas in a time-separated manner to one detector after the other. The third is to divide the gas flow and to lead the different branch flows to the different detectors in parallel.

An analyzer using the second possibility is described in EP-A1 0 024 566. It is used for measuring gas which evaporates from a material being heated in a crucible. A change-over valve is used to first measure with the first detector and then to measure afterwards with the second detector.

An analyzer using the first possibility is known from GB A 2 119 088. Said analyzer comprises the following features:

– a sample gas introducing passage;
– a diluting gas introducing passage;
– a sample gas flow dividing/diluting means connected to said introducing passages and having at least two exit passages;
and
– at least two gas concentration detectors, each one being connected to a respective exit passage.

The sample gas flow dividing/diluting means first branches the sample gas. To the branch flows diluting gas is added to such an extent that the concentration of a predetermined gas is the same in all branches, i.e. when the sample gas flow is decreased in a branch, the diluting gas flow is decreased by the same extent.

Using an analyzer of the first possibility has the disadvantage that only one fixed dilution ratio can be adjusted at a time. This makes it difficult to measure different gases of very different concentrations. Another problem is that wrong measurements may be induced by backpressure effects from one detector to another detector arranged before said detector. When using a gas analyzer of the third possibility, measurement is very timeconsuming as one ingredient has to be measured after the other. Using a gas analyzer of the third possibility has the disadvantage that a very low gas flow is obtained when choosing the branching ratio low for measuring the concentration of a very concentrated ingredient.

It is an object of the present invention to provide a gas analyzer for simultaneous highly accurate measurement of a plurality of ingredients contained in a sample gas mixedly comprising at least one high-concentration ingredient gas and one low-concentration ingredient gas.

The gas analyzer according to the present invention has the features of the analyzer using the third possibility as described above whereby said sample gas flow dividing/diluting means comprises at least two flow-dividing ratio/diluting ratio setting means for automatically reducing – or increasing – the dilution ratio when increasing – or decreasing, respectively – the flow dividing ratio. Therefrom, the advantage arises that the exit gas flow keeps high even when the sample gas flow is reduced. Preferably the dilution ratio is increased (reduced) to such an extent that the gas flow in the exit passage remains constant when the flow dividing ratio is decreased (increased), provided the pressures in the introducing passages remain constant.

Various possibilities exist to realize a flow dividing ratio/diluting ratio setting means having the just mentioned features. However, it is a special advantage to combine the mentioned functions in one single device. Such a device comprises a plurality of parallel passages, the entrances thereof being connectable exclusively either to the sample gas introducing passage or the diluting gas passage, the exit thereof being commonly connected to a respective exit passage. Due to the exclusive switching, always the same number of passages is used, each passage receiving either sample gas or diluting gas. Thereby automatically the feature is realized that the dividing ratio changes inversely to the diluting ratio.

Preferably, an ingredient previous rough concentration detector and a controller are used to supply adjusting values to said flow dividing ratio/diluting ratio setting means.

Further details of the invention and advantageous features thereof will be described in the following with reference to the accompanying drawings.

Fig. 1 is a schematic drawing of the basic concept of the present invention, comprising combined flow dividing ratio/diluting ratio setting means;

Fig. 2 a general view of the gas analyzer comprising a preferred embodiment of flow dividing ratio/diluting ratio setting means;

Fig. 3(A), (B) are diagrams showing the state of the principle members of the setting means for explaining various kinds of applications;

Fig. 4 is a general view showing an embodiment similar to the embodiment of Fig. 2, however, comprising an ingredients previous rough concentration detector; and

Fig. 5 is a concentration diagram showing a setting means comprising a plurality of three-way valves.

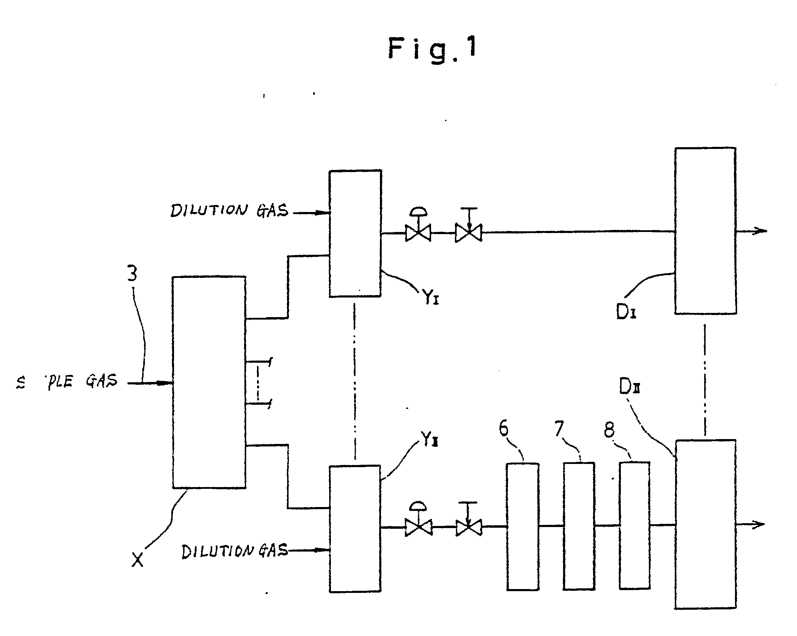

The analyzer according to Fig. 1 comprises a plurality of gas concentration detectors $D_I$, $D_{II}$, etc. connected in parallel to a sample gas-introducing passage provided with sample gas flow-dividing means X which can divide said sample gas in an appointed ratio to feed said gas concentration detectors $D_I$, $D_{II}$ with the divided flows of said sample gas, and sample gas diluting means $Y_I$, $Y_{II}$ for further diluting the sample gas divided by said sample gas-dividing means. The functions of the sample gas dividing means X and the sample gas diluting means $Y_I$, $Y_{II}$ are coupled in such a way that the diluting ratio is automatically increased in a branch when the dividing ratio for said branch is decreased, and inversely. Fig. 1 additionally shows that the following devices are arranged before the second detector D$_{II}$: an oxidyzer 6, a CO$_2$ gas remover 7, and a H$_2$O remover 8.

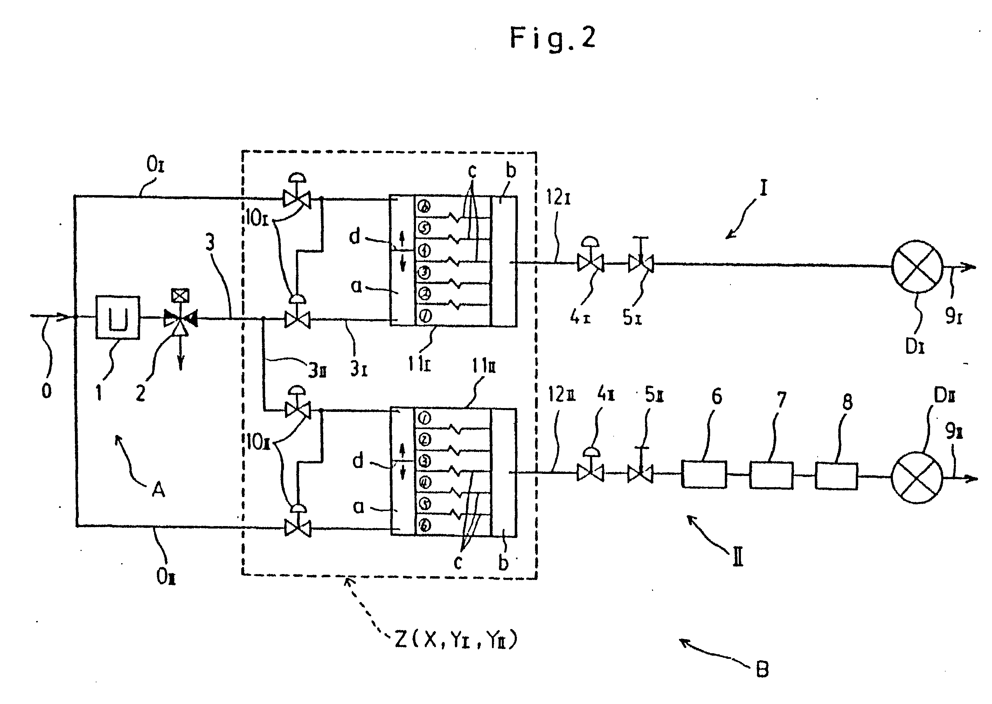

Fig. 2 is a general rough view showing a gas analyzer comprising combined flow dividing ratio/diluting ratio setting means.

This gas extraction type sample analyzer comprises, as shown in Fig. 2, a gas extraction portion A and a gas-analyzing portion B connected with said gas extraction portion A for simultaneously measuring two ingredients.

Said gas extraction portion A comprises a heating furnace 1 (for example a graphite crucible) for extracting various kinds of ingredient gases (CO gas, N$_2$ gas, H$_2$ gas and the like) corresponding to various kinds of ingredients (in this embodiment oxygen, nitrogen, hydrogen and the like) by heating it in the presence of a carrier gas (an inert gas such as He gas) supplied via a carrier gas-introducing passage O to the melt in the furnace 1 and an electromagnetic purge valve 2.

Said gas-analyzing portion B comprises a CO concentration measuring system I and a N$_2$ concentration measuring system II connected in parallel to a sample gas-introducing passage 3 connected with said electro-magnetic purga valve 2 of said gas extraction portion A.

Said CO concentration measuring system I comprises a first sample gas passage 3$_I$ branched from said sample gas-introducing passage 3, a first carrier gas passage O$_I$ branched from said carrier gas-introducing passage O, a first pressure-regulating governer 10$_I$ arranged between said first branched sample gas passage 3$_I$ and said first carrier gas passage O$_I$, a first flow-dividing ratio/diluting ratio-setting device 11$_I$ connecting said first branched sample gas passage 3$_I$ and said first carrier gas passage O$_I$, a first pressure regulator 4$_I$, a first flow rate-adjusting needle valve 5$_I$, a first gas concentration detector D$_I$ comprising a non-dispersion type infrared detector for use in measuring of the concentration of CO gas provided via a first exit passage 12$_I$ from said first flow-dividing ratio/diluting ratio-setting device 11$_I$ and a first exhaust passage 9$_I$.

In addition, said N$_2$ measuring system II comprises a second sample gas passage 3$_{II}$ branched from said sample gas-introducing passage 3, a second carrier gas passage O$_{II}$ branched from said carrier gas-introducing passage O, a second pressure-regulating governor 10$_{II}$ arranged between said second sample gas passage 3$_{II}$ and said second carrier gas passage O$_{II}$, a second flow-dividing ratio/diluting ratio-setting devide 11$_{II}$ connecting said second branched sample gas passage 3$_{II}$ and said second carrier gas passage O$_{II}$, a second pressure regulator 4$_{II}$, a second flow rate-adjusting needle valve 5$_{II}$, an oxidizer 6 for oxidizing CO gas and H$_2$ gas contained in the sample gas, which passed through first gas concentration detector D$_I$, to

be converted to CO$_2$ gas and H$_2$O gas, a CO$_2$ remover 7 for removing the resulting CO$_2$ gas by the chemical reaction between it and a CO$_2$ removing agent, a H$_2$O remover 8 for removing the resulting H$_2$O gas by a H$_2$O adsorbent, a second gas concentration detector D$_{II}$ comprising a heat conductivity type detector for use in measuring of the concentration of N$_2$ gas provided via a second exit passage 12$_{II}$ from said second flow-dividing ratio/diluting ratio-setting device 11$_{II}$ and a second exhaust passage 9$_{II}$.

In addition, a portion encircled by a dotted line in Fig. 2, mainly comprising said first flow-dividing ratio/diluting ratio-setting devide 11$_I$ and said second flow-dividing ratio/diluting ratio-setting device 11$_{II}$ shows a sample gas flow-dividing/diluting means Z which can be used both as a sample gas flow-dividing means X and a sample gas-diluting means Y$_I$, Y$_{II}$ described in Fig. 1 which is a schematic diagram of the basic concept of the present invention. Said sample gas flow-dividing/diluting means Z is adapted to divide a flow of the sample gas supplied through said sample gas-introducing passage 3 at an appointed ratio dilute the divided sample gas, and supply the first gas concentration detector D$_I$ and the second gas concentration detector D$_{II}$ arranged in parallel to said sample gas-introducing passage 3 with the divided and diluted sample gas. In other words, said sample gas-diluting means Y$_I$, Y$_{II}$ comprises the flow-dividing ratio/diluting ratio-setting means 11$_I$, 11$_{II}$, respectively, so as to be used in the cooperation also as said sample gas flow-dividing means X.

That is to say, each of said flow-dividing ratio/diluting ratio-setting means 11$_I$, 11$_{II}$ comprises an inlet side space a provided by said branched sample gas passage 3$_I$ (3$_{II}$) connected therewith at one end side thereof and said carrier gas passage O$_I$ (O$_{II}$) connected therewith at the other end side thereof, an outlet side space b from which said exit passage 12$_I$ (12$_{II}$) extends, a plurality (5 pieces in this embodiment) of flow rate elements (for example capillaries) communicating with said inlet side space a with said outlet side space b designated by c ----, and a valve body d dividing said inlet side space a into a space of the side at which said branched sample gas passage 3$_I$ (3$_{II}$) is connected and a space connected with said carrier gas passage O$_I$ (O$_{II}$). Said plurality of flow rate elements (capillaries) c---- used in this embodiment all have the same flow rate characteristics. In addition, the carrier gas (He gas) introduced through said carrier gas passage O$_I$ (O$_{II}$) is used as a diluting gas in said flow-dividing ratio/diluting ratio-setting device 11$_I$ (11$_{II}$). Said valve body d of said inlet side space a is adapted to be positioned at optional positions marked in the drawing with (1), (2), (3), (4), (5), (6), respectively. In other words, with each of said flow-dividing ratio/diluting ratio-setting devices 11$_I$ (11$_{II}$) it is optionally possible to adjust the flow-dividing ratio of the sample gas for each of said measuring systems I, II (said detectors D$_I$, D$_{II}$) as well as the diluting ratio of the divided sample gas by setting optionally the position of said valve body d. Besides, when the flow-dividing ratio is set to be large in each of said flow-dividing ratio/diluting ratio-setting devices $11_I$ ($11_{II}$), the diluting ratio is automatically set to be small (accordingly, the total dilution extent is small) while when the flow-dividing ratio is set to be small, the diluting ratio is automatically set to be large (accordingly, the total dilution extent is large). For example, under the condition as shown in Fig. 2, since the valve body d of the first flow-dividing ratio/diluting ratio-setting device $11_I$ is positioned at the position (4), the sample gas of a quantity corresponding to three pieces of flow rate elements c - - - and the carrier gas of a quantity corresponding to two pieces of flow rate elements c --- are introduced into the outlet side space b of the first flow-dividing ratio/diluting ratio-setting device $11_I$ while, since the valve body d of the second flow-dividing ratio/diluting ratiosetting device $11_{II}$ is positioned at the position (3), the sample gas of a quantity corresponding to two pieces of flow rate elements and the carrier gas of a quantity corresponding to three pieces of flow rate elements c - - - are introduced into the outlet side space b of the second flow-dividing ratio/diluting ratio-setting device $11_{II}$. Accordingly, the sample gas introduced through said sample gas-introducing passage 3 is transferred to the flow-dividing ratio/diluting ratio-setting devices $11_I$, $11_{II}$ at a flow-dividing ratio of 3 : 2, and the sample gas transferred to the first flow-dividing ratio/diluting ratio-setting device $11_I$ is diluted at a diluting ratio of 3/5 to be introduced into the measuring system I while the sample gas transferred to the second flow-dividing ratio/diluting ratio-setting device $11_{II}$ is diluted at a diluting ratio of 2/5 to be introduced into the measuring system II.

With a gas extraction type sample analyzer constructed in the above described manner, the following wide range of a sample gas can be tested with various kinds of measurements.

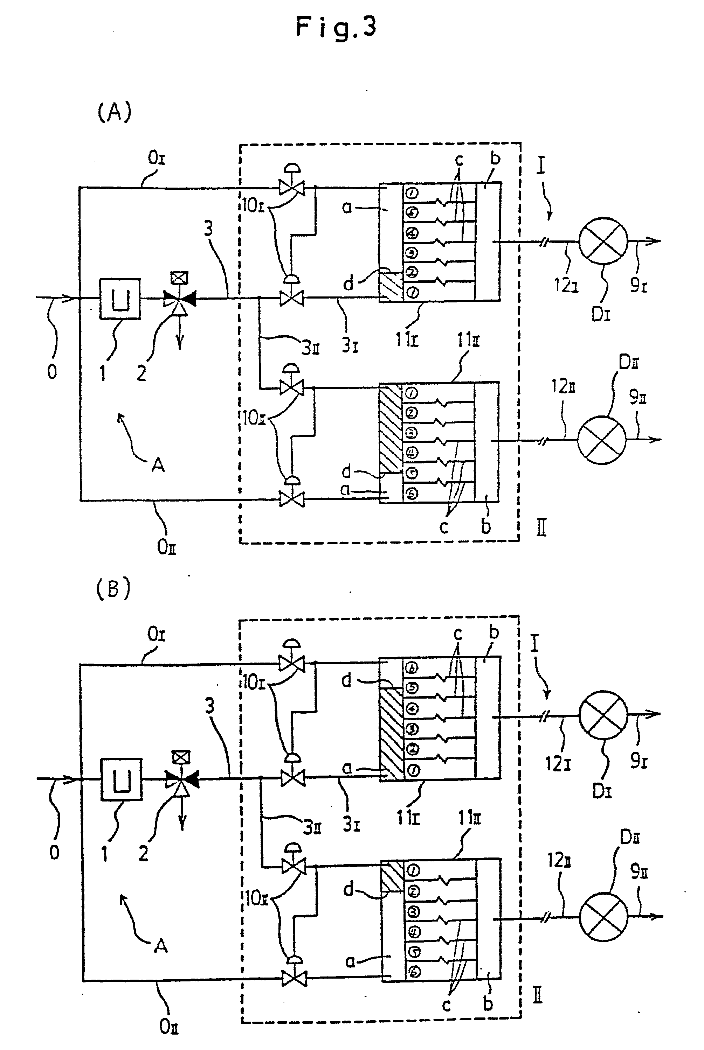

For example, in the case, where a sample, such as an oxide e.g. $Fe_2O_3$, comprising a large quantity of oxygen (principal ingredients) and a remarkably small quantity of nitrogen (impurities) is analyzed, the valve body d of the first flow-dividing ratio/diluting ratio-setting device $11_I$ is set to a certain position, for example the position (2), and the valve body d of the second flow-dividing ratio/diluting ratio-setting device $11_{II}$ is set to another position, for example the position (5), as shown in Fig. 3(A), whereby the sample gas introduced through the sample gas-introducing passage 3 is transferred to the first flow-dividing ratio/diluting ratio-setting device $11_I$ and the second flow-dividing ratio/diluting ratio-setting device $11_{II}$ at a flow-dividing ratio of 1 : 4, and the sample gas transferred to the first flow-dividing ratio/diluting ratio-setting device $11_I$ is diluted at a small diluting ratio of 1/5, while the sample gas transferred to the second flow-dividing ratio/diluting ratio-setting device $11_{II}$ is diluted at a large diluting ratio of 4/5, whereby the sample gas diluted at a remarkably large diluting extent is introduced into the CO concentration-measuring system I corresponding to a large quantity of oxygen, which is a principal ingredient, while the sample gas diluted at a rather small diluting extent is introduced into the $N_2$ concentration-measuring system II corresponding to a small quantity of nitrogen (impurities).

Also, in the case, where a sample, such as a silicide, e.g. $Si_3N_4$, comprising a large quantity of nitrogen (principal ingredient) and a very small quantity of oxygen (impurities) is analyzed, the valve body d of the first flow-dividing ratio/diluting ratio-setting device $11_I$ is positioned for example at the position (5), and the valve body d of the second flow-dividing ratio/diluting ratio-setting device $11_{II}$ is set to be positioned for example at the position (2), as shown in Fig. 3(B), whereby the sample gas introduced through the sample gas-introducing passage 3 is transferred to the flow-dividing ratio/diluting ratio-setting devices $11_I$, $11_{II}$ at a flow-dividing ratio of 4:1 and the sample gas transferred to the first flow-dividing ratio/diluting ratio-setting device $11_I$ is diluted at a large diluting ratio of 4/5, while the sample gas transferred to the second flow-dividing ratio/diluting ratio-setting device $11_{II}$ is diluted at a small diluting ratio of 1/5, whereby the sample gas diluted at a rather small diluting extent is introduced into the CO concentration-measuring system I corresponding to very small quantities of oxygen which are impurities, while the sample gas diluted at a remarkably large diluting extent is introduced into the $N_2$ concentration-measuring system II corresponding to a large quantity of nitrogen which is a principal ingredient in this case.

Furthermore, in the case, where a sample, such as metals, comprising a remarkable small but almost equal quantity of impurities such as oxygen and nitrogen, is analyzed, both the valve body d of the first flow-dividing ratio/diluting ratio-setting device $11_I$ and that of the second flow-dividing ratio-setting device $11_{II}$ are set to be positioned for example at the position (6), whereby the sample gas introduced through the sample gas-introducing passage 3 is transferred to the flow-dividing ratio/diluting ratio-setting devices $11_I$, $11_{II}$ at a flow-dividing ratio of 1:1, and both the sample gas transferred to the first flow-dividing ratio/diluting ratio-setting device $11_I$ and the sample gas transferred to the second flow-dividing ratio/diluting ratio-setting device $11_{II}$ are diluted at a diluting ratio of 0/5, that is to say the sample gas flow is divided but not diluted.

In addition, according to the sample gas to be measured, the valve body d of the first flow-dividing ratio/diluting ratio-setting device $11_I$ and the valve body d of the second flow-dividing ratio/diluting ratio-setting device $11_{II}$ can optionally be positioned at any suitable position such that many ingredients contained in a wide range of samples having various ingredient gas concentration distributions can be simultaneously measured with high accurracy by means of a single apparatus.

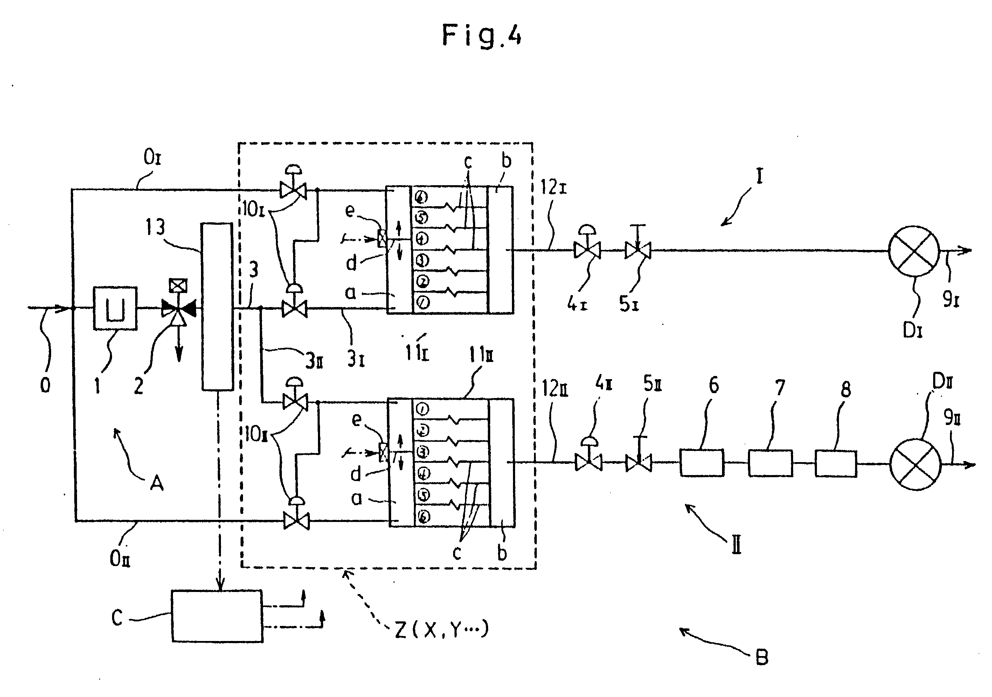

Fig. 4 shows another preferred embodiment in which the sample gas-introducing passage 3 is provided with a previous concentration detector 13 for previously detecting rough concentrations of ingredients contained in a sample gas introduced therein, and the valve bodies d of the flow-dividing ratio/diluting ratio-setting devices $11_I$, $11_{II}$ are provided with actuating mechanisms e, respectively, whereby the valve bodies d is a automatically set to be positioned at the suitable position by means of a controller C on the basis of a rough concentration ratio of ingredient gases detected by said previous concentration detector 13. Other members are similar to those in the above described embodiment.

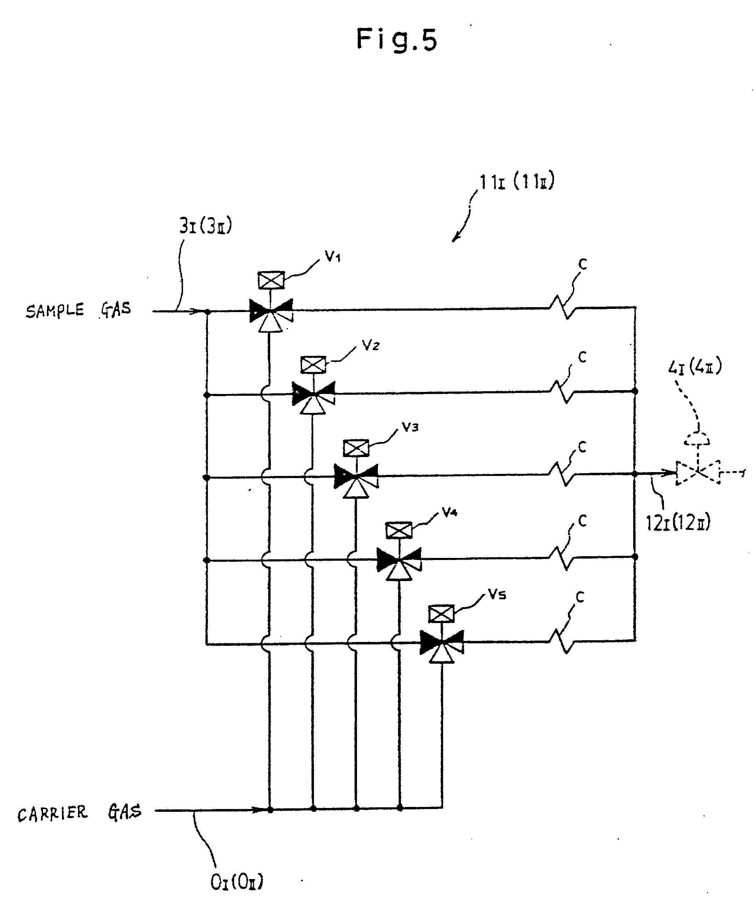

Fig. 5 shows another preferred embodiment of the flow-dividing ratio/diluting ratio-setting device $11_I$ ($11_{II}$) comprising a plurality (five in this embodiment) of electro-magnetic three-way valves $v_1$, $v_2$ — into which the branched passages from the branched sample gas passage $3_I$ ($3_{II}$) and the branched passages from the carrier gas passage $O_I$ ($O_{II}$) are opening, and flow rate elements (for example capillaries) c, — connected with said electro-magnetic three-way valves $v_1$, $v_2$ —. As can be recognized by the expert, the flow-dividing ratio/diluting ratio-setting devices $11_I$, $11_{II}$ of such a construction provide for the same advantageous results as that of the flow-dividing ratio/diluting ratio-setting devices $11_I$, $11_{II}$ of the above described embodiments.

Although a gas analyzer capable of simultaneously measuring two ingredients was described by embodiments, it is within the scope of the present invention to also provide a gas analyzer for simultaneous measurement of many ingredients, i. e. for example more than three ingredients, as indicated in Fig. 1 which is a general sketch of the concept of the present invention.

In addition, although a sample gas flow-dividing/diluting means Z, in which a sample gas flow-dividing means X and sample gas-diluting means $Y_I$, $Y_{II}$ are collected, has been described with the above, the expert may readily realize that the sample gas flow-dividing means X and the sample gas-diluting means $Y_I$, $Y_{II}$ may be constructed as independent units.

## Claims

1. A gas analyzer for the simultaneous measurement of a plurality of ingredients, that analyzer comprising
   - a sample gas introducing passage (3);
   - a diluting gas introducing passage (0);
   - a sample gas flow dividing/diluting means (Z) being connected to said introducing passages and having at least two exist passages ($12_I$, $12_{II}$), said means (Z) adjusting a flow dividing ratio and diluting ratios, wherein the flow dividing ratio is the ratio of sample gas flows through the two exit passages, and each diluting ratio is the ratio between the overall gas flow through one exit passage and the sample gas flow through said passage; and
   - at least two concentration detectors ($D_I$, $D_{II}$), each one being connected to a respective exit passage, characterized in that
   - said sample gas flow dividing/diluting means comprises at least two flow dividing ratio/diluting ratio setting means ($11_I$, $11_{II}$) being arranged to automatically increase or decrease the flow dividing ratio when the diluting ratio is decreased or increased, respectively, in one of said setting means.

2. The gas analyzer according to claim 1, characterized in that each flow dividing ratio/diluting ratio setting means automatically reduces the dilution ratio to such an extent that, when increasing the flow dividing ratio, the gas flow in the exit passage remains constant, provided the pressures in the introducing passages remain constant, or inversely.

3. The analyzer according to claim 2, characterized in that each flow dividing ratio/diluting ratio setting means comprises a plurality of parallel passages (c), the entrances thereof being connectable exclusively either to the sample gas introducing passage (3) or the diluting gas introducing passage (0).

4. The gas analyzer according to one of the claims 1–3, characterized by an ingredients previous rough concentration detector (13) and a controller (C) which is adapted to receive the detected values from said ingredients previous rough concentration detector (13) and to supply adjusting values to said flow dividing ratio/diluting ratio setting devices ($11_I$, $11_{II}$).

## Patentansprüche

1. Gasanalysator für die gleichzeitige Messung mehrerer Substanzen mit
   - einer Probengas-Einlaßleitung (3);
   - einer Verdünnungsgas-Einlaßleitung (0);
   - einer Einrichtung (Z) zur Flußaufteilung/Verdünnung des Probengases, die mit den Einlaßleitungen verbunden ist und mindestens zwei Ausgangsleitungen ($12_I$, $12_{II}$) aufweist, und ein Verhältnis der Flußaufteilung/Verdünnung einstellt, wobei das Verhältnis der Flußaufteilung dem Verhältnis der Probengasflüsse durch die zwei Ausgangsleitungen entspricht und das Verdünnungsverhältnis dem Verhältnis zwischen dem gesamten Gasfluß durch eine der Auslaßleitungen und dem Probengasfluß durch diese Leitung entspricht; und
   - mindestens zwei Konzentrationsdetektoren ($D_I$, $D_{II}$), von denen jeder mit einer jeweiligen Auslaßleitung verbunden ist, dadurch gekennzeichnet, daß
   - die Einrichtung (Z) zur Aufteilung des Probengases bzw. der Verdünnung mindestens zwei Einstellmittel ($11_I$, $11_{II}$) für das Verhältnis der Flußaufteilung/Verdünnung aufweist, die so angeordnet sind, daß das Flußaufteilungsverhältnis automatisch erhöht oder erniedrigt wird, wenn das Verdünnungsverhältnis in einem der Einstellmittel erniedrigt bzw. erhöht wird.

2. Gasanalysator nach Anspruch 1, dadurch gekennzeichnet, daß jedes der Einstellmittel für das Verhältnis der Flußaufteilung/Verdünnung das Verdünnungsverhältnis automatisch so weit verringert, daß dann, wenn das Flußaufteilungsverhältnis zunimmt, der Gasfluß unter der Voraussetzung konstanter Drücke in den Eingangsleitungen konstant bleibt bzw. umgekehrt.

3. Gasanalysator nach Anspruch 2, dadurch gekennzeichnet, daß jedes der Einstellmittel für das Verhältnis der Flußaufteilung/Verdünnung mehrere parallele Leitungen (c) aufweist, deren Eingänge ausschließlich entweder mit der Probengas-Einlaßleitung (3) oder der Verdünnungsgas-Einlaßleitung (0) verbindbar sind.

4. Gasanalysator nach einem der Ansprüche 1 bis 3, gekennzeichnet durch einen Detektor (13) zur groben Vorausbestimmung von Substanzkonzentrationen und durch eine Steuereinrichtung (C), die die vom Detektor (13) zur groben Vorausabstimmung von Substanzkonzentrationen ermittelten Werte aufnimmt und Einstellwerte an die Einstellmittel (11I, 11II) für das Verhältnis der Flußaufteilung/Verdünnung liefert.

## Revendications

1. Analyseur de gaz pour la mesure simultanée d'une pluralité d'ingrédients, comportant
- un passage d'introduction (3) d'un gaz échantillon ;
- un passage d'introduction (0) de gaz de dilution;
- un organe de division d'écoulement de gaz / dilution (Z) étant connecté auxdits passages d'introduction et possédant au moins deux passages de sortie (12I, 12II), ledit organe (Z) réglant un rapport de division d'écoulement et des rapports de dilution, dans lequel le rapport de division d'écoulement est le rapport des débits de gaz échantillon à travers les deux passages de sortie, et chaque rapport de dilution est le rapport entre le débit global du gaz à travers un passage de sortie et le débit de gaz échantillon à travers ledit passage ; et
- au moins de deux détecteurs de concentration ($D_I$, $D_{II}$), connectés chacun à un passage de sortie respectif, caractérisé en ce que
- ledit organe de division d'écoulement de gaz échantillon/ dilution comporte au moins deux organes de réglage du rapport de division d'écoulement/rapport de dilution (11I, 11II) agencés pour augmenter ou diminuer automatiquement le rapport de division d'écoulement lorsque le rapport de dilution est diminué ou augmenté, respectivement, dans l'un desdits moyens de réglage.

2. Analyseur de gaz selon la revendictaion 1, caractérisé en ce que chaque organe de réglage du rapport de division d'écoulement/rapport de dilution réduit automatiquement le rapport de dilution dans une mesure telle que, lorsque le rapport de division d'écoulement augmente, le débit de gaz dans le passage de sortie demeure constant, pourvu que les pressions dans les passages d'introduction demeurent constantes ou inversement.

3. Analyseur de gaz selon la revendication 2, caractérisé en ce que chaque organe de réglage du rapport de division d'écoulement/rapport de dilution comporte une pluralité de passages parallèles (c), dont les entrées peuvent être reliées exclusivemen, soit au passage d'introduction de gaz échantillon (3), soit au passage d'introduction de gaz de dilution.

4. Analyseur de gaz selon l'une quelconque des revendications 1 à 3, caractérisé par un détecteur de concentration grossière préalable des ingrédients (13) et un organe de commande (C) qui est agencé pour recevoir les valeurs détectées en provenance dudit détecteur de concentration grossière préalable des ingrédients (13) et pour délivrer des valeurs de réglage auxdits dispositifs de réglage de rapport de division d'écoulement/rapport de dilution (11I, 11II).

Fig.1

# Fig.2

EP 0 222 994 B1

# Fig.3

(A)

(B)

Fig.4

EP 0 222 994 B1

Fig.5